# EUROPEAN PATENT APPLICATION

(11) **EP 4 008 408 A1**
(43) Date of publication of application: **08.06.2022**
(21) Application number: 21192351.1
(22) Date of filing: 20.08.2021
(51) Int. Cl.: A62B 18/08, A41D 13/11, A62B 23/02

(54) **BREATHING GUIDE APPARATUS AND MASK SYSTEM INCLUDING THE SAME**

(30) Priority: 01.12.2020 KR 20200165475; 14.04.2021 KR 20210048230
(71) Applicant: Lee, In Bok, Yongin-si, Gyeonggi-do 16802 (KR)
(72) Inventor: Lee, In Bok, Yongin-si, Gyeonggi-do 16802 (KR)
(74) Representative: advotec.

(57) **Abstract**

The present invention relates to a breathing guide apparatus readily mountable inside an air filtration mask for filtering foreign substances contained in the air, such as fine dust, pathogens, viruses, and dust, and configured to separate an exhalation path of a user of a mask from his or her inhalation path and exhaust the user's exhalation to the outside, thereby reducing suffocating or discomfort due to carbon dioxide or bad breath in the exhalation remaining in the mask, limit the user's exhalation exhaust path to the breathing guide apparatus when the user is wearing glasses to minimize dew condensation on the glasses due to moisture contained in the exhalation, and double-filter pathogens, viruses, or bad breath during exhaust of an exhalation of a user infected with or suspected to be infected with an infectious disease, thereby minimizing the spread of the infectious disease or a third party's anxiety; and a mask system including the same.

## Description

### FIELD OF THE INVENTION

The present invention relates to a breathing guide apparatus and a mask system including the same. More specifically, the present invention relates to a breathing guide apparatus readily mountable inside an air filtration mask for filtering foreign substances contained in the air, such as fine dust, pathogens, viruses, and dust, and configured to separate an exhalation path of a user of a mask from his or her inhalation path and exhaust the user's exhalation to the outside, thereby reducing suffocating or discomfort due to carbon dioxide or bad breath in the exhalation remaining in the mask, limit the user's exhalation exhaust path to the breathing guide apparatus when the user is wearing glasses to minimize dew condensation on the glasses due to moisture contained in the exhalation, and double-filter pathogens, viruses, or bad breath during exhaust of an exhalation of a user infected with or suspected to be infected with an infectious disease, thereby minimizing the spread of the infectious disease or a third party's anxiety; and a mask system including the same.

### BACKGROUND ART

Generally, a mask is a product that covers respiratory organs such as a nose and mouth, and is configured to block dust or foreign substances in the air from being inhaled into the respiratory organs of a user of the mask and to prevent saliva or bad breath secreted from the user's respiratory organs from being scattered and spread. With the increasing of yellow dust or fine dust and the emergence of high-infectious new viruses, masks with improved performance of filtering both inhalation and exhalation are in demand.

However, when a user exhales through a mask with a filter, the user's exhalation is likely to remain inside the mask due to filter resistance or the like, and thus, the user may inhale carbon dioxide and feel suffocating and discomfort when inhaling through the mask in which carbon dioxide or bad breath remains and may experience dew condensation when the user is wearing glasses. Therefore, the user may be reluctant to use the mask.

In order to solve difficulties with the exhaust of exhalation due to filter resistance, a mask in which a filter unit is partially cut and an exhaust valve is additionally provided to exhaust non-filtered exhalation has been introduced. However, when a user of the mask is a patient infected with or suspected to be infected with an infectious disease, the spread of the infectious disease may be promoted when the user's exhalation is exhausted without being filtered and thus this mask is not desirable. In the case of a recent epidemic of an infectious virus disease, it was found that the disease is very contagious even during an incubation period of the virus and thus it is not desirable to exhaust non-filtered exhalation of a user of the mask. There are cases in which wearing a mask with an exhaust valve configured to exhaust non-filtered exhalation is prohibited by law.

### SUMMARY OF THE INVENTION

According to an aspect of the present invention, there is provided a breathing guide apparatus mounted inside a filter unit of a mask for air filtration, the breathing guide apparatus comprising: an exhalation vent member configured to be disposed in front of the mouth of a user of the mask to exhaust an exhalation from the user's mouth; at least one exhalation path member connected to the exhalation vent member and extending to a left or right side of the exhalation vent member; and at least one exhalation exhaust member configured to cause the exhalation to pass through the inner circumferential surface of the filter unit, filter foreign substances contained in the exhalation, and exhaust the resultant exhalation, the at least one exhalation exhaust member being provided on an end of the exhalation path member to be in close contact with an inner circumferential surface of the filter unit.

And the exhalation exhaust member may be brought in close contact with a left or right inner circumferential surface of the filter unit of the mask to cause the user's inhalation to pass through the filter unit of the mask and be inhaled into the user's nose' and to cause the user's exhalation to pass through the filter unit of the mask and be exhausted without being mixed with the inhalation.

And a pair of exhalation path members and a pair of exhalation exhaust members may be provided to be connected to the left side and a right side of the exhalation vent member.

And the exhalation exhaust member may be provided in the form of a concave bowl, and the exhalation path member is connected to a lateral side of the exhalation exhaust member.

And the exhalation path member may be mounted to pass through the exhalation exhaust member.

And an end of the exhalation path member may be inclined to prevent interference with the inner circumferential surface of the filter unit of the mask, while being mounted to pass through the exhalation exhaust member.

And the exhalation exhaust member may tapers in a direction opposite to a connection portion thereof to which the exhalation path member is connected.

And an exhaust port of the exhalation exhaust member may be curved backward to be in close contact with the filter unit of the mask.

And both ends of the exhalation path member may be provided with ring-shaped bumps in a circumferential direction to prevent separation of the exhalation path member or seal the exhalation path member, when fastened with the exhalation vent member and the exhalation exhaust member.

And the exhalation path member may be formed of a polyvinyl chloride (PVC) material, polypropylene (PP), polyethylene (PE) or a silicon material, and the exhalation vent member or the exhalation exhaust member is formed of a PVC material, PP, PE, or a silicon material.

And an edge of an exhaust port of the exhalation exhaust member may be provided with a plurality of fixing pins to fix the inner circumferential surface of the filter unit of the mask.

And the breathing guide apparatus may further comprise an exhaust port filter configured to filter an exhalation to be exhausted through the exhaust port of the exhalation exhaust member.

And the exhalation vent member may comprise a vent port configured to be placed in front of the user's mouth; and at least vent port configured to deliver an exhalation vent through the vent port to the exhalation path member.

And at least a region of the exhalation path member may have a height greater than a thickness of a cross section thereof.

And the exhalation vent member, the exhalation path member, and the exhalation exhaust member of the breathing guide apparatus may be separate component.

And the exhalation path member may be mounted in the exhalation vent member or the exhalation exhaust member to be insertable into or withdrawable from the exhalation vent member or the exhalation exhaust member, and thus, a distance between the exhalation exhaust member and the exhalation vent member and an angle of connection to the exhalation path member may be adjustable.

And the exhaust port of the exhalation exhaust member may be further provided with a plurality of fixing pins to pass through the filter unit of the mask, and the breathing guide apparatus may further comprise a fixing member formed in the form of a ring or a cap provided with a fixing hole or a fixing groove into which the fixing pins, which pass through the filter unit of the mask and protrude from a front surface of the filter unit, are inserted and fixed.

And the breathing guide apparatus may further comprise wearable bands configured to be mounted on the fixing member to be hung on the user's ears, when the fixing member is formed in the form of the cap.

And when the fixing member may be formed in the form of the cap, an exhaust port is formed on a lateral side or a lower side of an edge of the fixing member.

And the exhalation vent member may have a chamber type body, wherein the exhalation vent member may comprises a vent port configured to be disposed in front of a mouth of a user of the mask; at least one exhaust port configured to deliver exhalation exhausted via the vent port to the exhalation path member; and a blocking plate configured to prevent contact between the filter unit of the mask and the user's lips.

### BRIEF DESCRIPTION OF THE DRAWINGS

FIG. 1 illustrates a state in which a user is wearing a mask system equipped with a breathing guide apparatus according to an embodiment of the present invention;
FIG. 2 is a top view of a state in which the breathing guide apparatus and a mask are taken off from a user of the mask system of FIG. 1 and are disposed apart from each other;
FIG. 3 illustrates a perspective view and an exploded perspective view of a breathing guide apparatus according to an embodiment of the present invention;
FIG. 4 is a perspective view of examples of a breathing guide apparatus according to an embodiment of the present invention;
FIG. 5 illustrates a state in which a user is wearing a mask system equipped with a breathing guide apparatus according to another embodiment of the present invention;
FIG. 6 illustrates various examples of a mask included in a mask system according to an embodiment of the present invention;
FIG. 7 illustrates a breathing guide apparatus according to another embodiment of the present invention;
FIG. 8 illustrates a breathing guide apparatus according to another embodiment of the present invention;
FIG. 9 illustrates a state in which a user is wearing a general disposable mask mainly used for dental or medical purposes;
FIG. 10 is a perspective view of a state in which a breathing guide apparatus according to another embodiment of the present invention is mounted in the mask of FIG. 9;
FIG. 11 illustrates a state in which a fixing member is separated from a breathing guide apparatus according to an embodiment of the present invention;
FIG. 12 illustrates a front and rear perspective views of a fixing member of a breathing guide apparatus according to an embodiment of the present invention; and
FIG. 13 illustrates a breathing guide apparatus according to another embodiment of the present invention.

### Advantageous Effects

A breathing guide apparatus according to the present invention is configured to be easily detachably attached to general-purpose filter type masks designed to filter foreign substances contained in the air, such as fine dust, pathogens, viruses, and dust, and thus can be mounted in almost all filter type masks and minimize inconvenience or discomfort that may be caused to a user of a mask while allowing functions of the mask to be fully used.

In the breathing guide apparatus according to the present invention, exhalation path members are provided at a left side and a right side of the mouth around which an exhalation ventilation member is disposed, so that inhalation flowing near the nose and exhalation to be exhausted via an exhalation exhaust member may be prevented from being mixed as much as possible.

In the breathing guide apparatus and a mask system including the same according to the present invention, a path through which exhalation is exhausted is separated and exhalation exhaust members are disposed at left and right sides of the exhalation ventilation member to be spaced apart from each other, thereby minimizing inconvenience due to condensation on glasses due to exhalation.

The breathing guide apparatus according to the present invention is configured such that the exhalation ventilation member is located in a concave space between a user's cheekbones and jawbone to prevent a filter unit of a mask from being lifted, thereby minimizing inflow of external air that does not pass through the filter unit or exhaust of exhalation that does not pass through the filter unit.

In the breathing guide apparatus and the mask system including the same according to the present invention, a great part of exhalation is exhausted to the outside through a path separated from that of inhalation due to the breathing guide apparatus, thereby reducing suffocating or discomfort due to carbon dioxide or bad breath remaining in a mask.

In the breathing guide apparatus and the mask system including the same according to the present invention, exhalation is exhausted through the breathing guide apparatus to limit a region of the filter unit of the mask, which is likely to be wet due to saliva or wet gas secreted from a user of the mask, to an exhaust port of the exhalation exhaust member, thereby minimizing a wet region of the filter unit from being in direct contact with the user's mouth or skin around the mouth.

In the breathing guide apparatus and the mask system including the same according to the present invention, a path through which exhalation is exhausted and a path through which inhalation is inhaled are separated from each other, and exhalation passes through the exhalation exhaust member, is filtered by the filter unit of the mask or an exhaust port filter of the exhaust port of the exhalation exhaust member, and is thereafter exhausted, thereby minimizing the spread of an infectious disease due to pathogens or virus contained in exhalation when a user of the mask has been infected with the infectious disease.

In addition, the exhalation exhaust member of the breathing guide apparatus is further provided with a plurality of fixing pins, which is configured to pass through the filter unit of the mask, to fix a position of the exhalation exhaust member, and a ring or cap type fixing member formed of an elastic material such as silicon or rubber and including through-holes or through-grooves into which the plurality of fixing pins, when passing through the filter unit of the mask and protruding from a front surface of the filter unit, are fixedly inserted, may be further provided to firmly mount the exhalation exhaust member on the filter unit of the mask.

The breathing guide apparatus according to the present invention may further include a cap type fixing member and a wearable band while the filter unit of the mask is interposed therebetween, so that the filter unit of the mask may be in close contact with each other, thereby minimizing the amount of exhalation exhausted without being filtered.

In addition, in the breathing guide apparatus according to the present invention, the exhalation ventilation member may include a blocking plate to prevent contact between the lips and the filter unit of the mask, thereby minimizing the transfer of saliva, virus, contaminants, etc. between the lips and the filter unit.

### DETAILED DESCRIPTION

Hereinafter, exemplary embodiments of the present invention will be described in detail with reference to the accompanying drawings. The present invention is, however, not limited thereto and may be embodied in many different forms. Rather, the embodiments set forth herein are provided so that this disclosure may be thorough and complete and fully convey the scope of the invention to those skilled in the art. Throughout the specification, the same reference numbers represent the same elements.

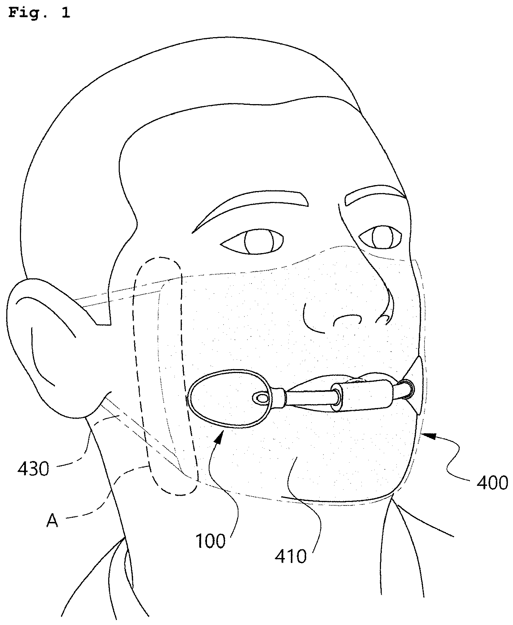

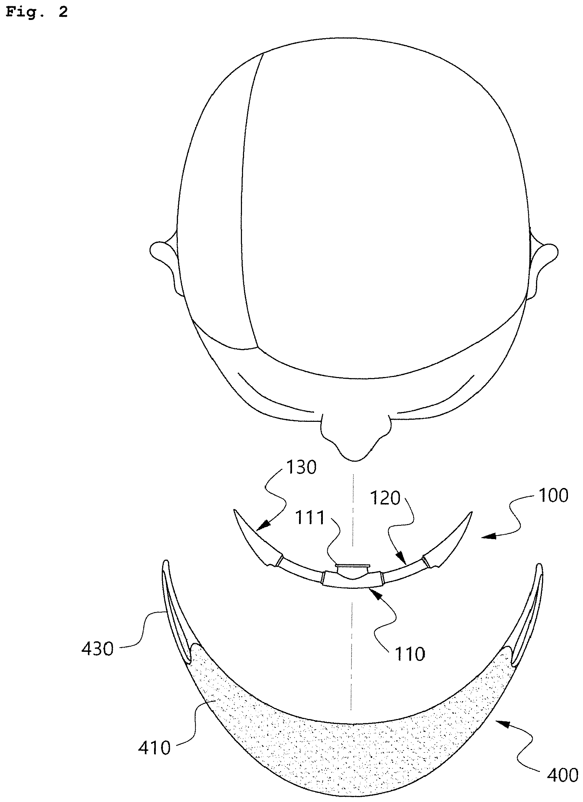

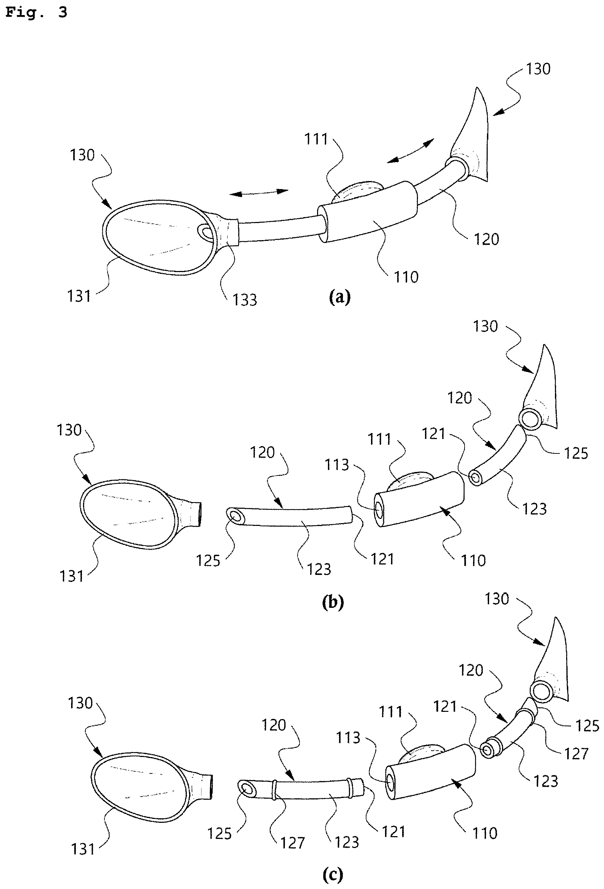

FIG. 1 illustrates a state in which a user is wearing a mask system equipped with a breathing guide apparatus according to an embodiment of the present invention. FIG. 2 is a top view of a state in which the breathing guide apparatus and a mask are taken off from the user who is wearing the mask system of FIG. 1 and are disposed apart from each other. FIG. 3 illustrates a perspective view and an exploded perspective view of a breathing guide apparatus according to an embodiment of the present invention.

As illustrated in FIGS. 1 to 3, a breathing guide apparatus 100 according to an embodiment of the present invention may be used by being mounted inside a filter unit 410 of a mask 400 for air filtration, and may include: an exhalation vent member 110 disposed in front of the mouth of a user of the mask 400 to exhaust exhalation, an exhalation flow path member 120 connected to the exhalation vent member 110 and extending to a left or right side of the filter unit 410, and at least one exhalation exhaust member 130 provided on an end of the exhalation flow path member 120 to be in close contact with an inner circumferential surface of the filter unit 410 to exhaust exhalation while passing through the inner circumferential surface of the filter unit 410.

In addition, the present invention provides a mask system including the breathing guide apparatus 100 and a mask 400 for air filtration. The mask 400 includes: the filter unit 410, for air filtration, having an inner circumferential surface to be in close contact with the exhalation exhaust member 130 of the breathing guide apparatus 100; and a wearable member 430 included in the filter unit 410 and configured to be hung on the ears or head of a user of the mask. The user may exhale through the breathing guide apparatus 100 and the filter unit 410 and inhale through a region of the filter unit 410 on which the breathing guide apparatus 100 is not mounted.

Recently, as air quality becomes worse due to fine dust or yellow dust and new viruses are spreading, the number of people who wears a mask to guard their health is increasing. In order to more effectively block fine particles in the air, the demand for masks having a high dust blocking rate (e.g., KF, N, or KN) is increasing, but the higher the dust blocking rate, the higher exhaust resistance with respect to exhalation.

The filter unit 410 of the mask 400 of the present invention may include a melt blown (MB) filter, a nano filter, an additional nonwoven filter, a functional filter, or a combination thereof and may be formed in about one to five layers.

If when the user exhales through a mask, the exhalation is not smoothly exhausted and remains in the mask, carbon dioxide or bad breath contained in the exhalation will be inhaled by the user when the user inhales through the mask.

In addition, when users who are wearing glasses exhale through a mask, a part of the exhalation may leak into a gap between the mask and the skin and thus dew condensation may occur on the glasses due to moisture contained in the leaking exhalation and a temperature difference between external air and the exhalation. Thus, the users will experience difficulties in securing a clear view. To solve these problems, a mask that does not include a filter but includes an exhaust valve to exhaust exhalation without exhaust resistance has been introduced. However, because virus or bacteria may be contained in exhalation of a patient infected with an infectious disease, such a mask equipped with an exhaust valve is not desirable in terms of public hygiene and wearing thereof is prohibited by law in some countries or regions.

In the breathing guide apparatus 100 and the mask system including the same according to the present invention, a user of the mask 400 may exhale through the breathing guide apparatus 100 inside the filter unit 410 of the mask 400 for air filtration, thereby preventing the user's exhalation and inhalation from being mixed.

Specifically, the exhalation vent member 110 may be disposed in front of the mouth of the user of the mask 400, the exhalation path member 120 may be connected to both sides of the exhalation vent member 110, and the exhalation exhaust member 130 may be provided on ends of the exhalation vent member 110. As illustrated in FIG. 3, when a user wears the mask 400, the exhalation vent member 110 is disposed in front of the user's mouth, the exhalation exhaust member 130 is brought into close contact with the filter unit 410 while being placed in cheek regions of the user's face, and particularly, concave regions of outer sides of back teeth between the user's cheekbones and jawbone. Thus, when the user of the mask 400 exhales, carbon dioxide and breathe may pass through the filter unit 410 corresponding to an inner circumferential surface of the exhalation exhaust member 130 via the exhalation vent member 110 and the exhalation path member 120 and thereafter be exhausted to the outside of the mask 400.

Generally, as shown in FIG. 1, the filter unit 410 of the mask 400 may extend upward to a space between the user's cheekbone and ears and extend downward to cover the user's left and right jaw bones, and the wearable member 430 may be configured to be flexibly fixed on the user's ears or the like, so that left and right end regions A of the filter unit 410 may be in close contact with the user's cheeks to prevent inflow of external air and leakage of exhalation.

Thus, the exhalation exhaust member 130 of the breathing guide apparatus 100 of the present invention may be disposed in the concave regions of the outer sides of the back teeth between the user's cheekbones and jawbone to be paced inside the end regions A in close contact with the skin, so that pain or feeling of irritation caused by interference between the exhalation exhaust member 130 and the cheekbones or the jawbone may be minimized and the filter unit 410 may be prevented from being lifted, thereby minimizing inflow of external air that does not pass through the filter unit 410 and leakage of exhalation that does not pass through the filter unit 410.

The exhalation vent member 110 may include a vent port 111 configured to be disposed in front of the mouth of a user of the mask 400 and at least one exhaust port 113 for delivering exhalation exhausted via the vent port 11 to the exhalation path member 120.

The vent port 111 of the exhalation vent member 110 may have stepped outer edges so that the breathing guide apparatus 100 may be easily bitten by a user for a short time when the user first wears the breathing guide apparatus 100.

Although not shown, the vent port 111 may be formed in a cone shape to collect exhalation as much as possible but may be formed in an inwardly recessed form or a ridge-and-furrow form to minimize interference with the lips.

The vent port 111 may be embodied in many different forms, provided that it is located in front of a user's lips to supply exhalation when the user is wearing the breathing guide apparatus 100.

The exhalation vent member 110 may be disposed in front of the user's mouth, extend to the vicinity of the lips to be in contact with the lips, or be configured in a mouthpiece shape to be bitten between the lips.

Here, the user may exhale through the exhalation vent member 110 in front of his or her mouth. As described above, because the exhalation vent member 110 is disposed in front of the user's mouth, most of exhalations may flow to the exhalation path member 120 connected to the exhalation vent member 110 and then be exhausted via the exhalation exhaust member 130.

The exhalation exhaust member 130 may be fixed in close contact with the inside of the filter unit 410 of the mask 400 by various fixing means as described below to block a gap between the exhalation exhaust member 130 and the filter unit 410, and exhalation flowing to the exhalation path member 120 through the exhalation vent member 110 may be exhausted through the exhalation exhaust member 130, thereby minimizing inflow of the exhalation into an inner space of the mask 400.

The breathing guide apparatus 100 may be configured such that the exhalation vent member 110 is disposed in front of the user's mouth and the exhalation path member 120 is connected to opposite ends of the exhalation vent member 110 in a horizontal direction, so that the exhalation exhaust member 130 may be brought into close contact with inner sides of left and right regions of the filter unit 410 that are spaced apart from each other.

Thus, the sum of the areas of the exhaust ports 131 of the pair of the exhalation exhaust members 130 is larger than the area of the vent port of the exhalation vent member 110, thereby minimizing exhaust resistance when exhalation is exhausted.

Not only carbon dioxide but also moisture, saliva, etc. may flow to the exhalation path member 120 via the exhalation vent member 110 in front of the user's mouth when the user exhales through the mask 400. Thus, moisture may be concentrated only in a region of the filter unit 410 corresponding to the exhaust port 131 of the exhalation exhaust member 130 and prevented from being in contact with the user's mouth or the skin near the mouth due to the breathing guide apparatus 100, thereby minimizing discomfort caused to the user.

Moisture or water continuously generated in a mask may be accompanied by bad smell but moisture or bad smell may be reduced due to the region of the filter unit 410 in which moisture is collected, i.e., a region of the mask 400 that is an inhalation region between the user's nose and mouth.

As illustrated in FIG. 3A, a pair of exhalation path members 120 and a pair of exhalation exhaust members 130 are provided to be respectively connected to a left side and a right side of the exhalation vent member 110. The exhalation vent members 130 may be configured in a concave bowl form and the exhalation path members 120 may be connected in a side direction of the exhalation exhaust members 130.

As shown in the exploded perspective view of FIG. 3B, the exhalation path member120 may be configured to be mounted while passing through the exhalation exhaust member 130.

That is, the breathing guide apparatus 100 may include one exhalation vent member 110, two exhalation path members 120, and two exhalation exhaust members 130 to be assembled together.

The exhalation path member 120 may be mounted through the exhalation exhaust member 130 or the exhalation substrate member 110, when assembled.

The exhalation exhaust member 130 may taper in a direction opposite to a connection portion thereof to which the exhalation path member 120 is connected, thereby minimizing generation of protruding or lifted regions, even when the exhalation exhaust member 130 is mounted in a mask.

According to the shape of the exhalation exhaust member 130, an inner end of the exhalation path member 120 to be passed through or inserted into the exhalation vent member 110 among both ends thereof is not limited in shape but an outer end thereof to be passed through the exhalation exhaust member 130 may be processed to be inclined or cut to prevent interference with the inner circumferential surface of the filter unit 410 of the mask 400.

The exhaust port 131 of the exhalation exhaust member 130 may be formed in a curved shape to be in close contact with the inner circumferential surface of the filter unit 410 of the mask 400, in consideration of the curves of the face.

As illustrated in FIGS. 3A and 3B, the exhalation vent member 110, the exhalation path member 120, and the exhalation exhaust member 130 may be separate components and be assembled together. Because the exhalation path member 120 is mounted to pass through the exhalation exhaust member 130 to be insertable into or withdrawable from the exhalation exhaust member 130. Thus, the exhalation exhaust member 130 may slide to a certain extent on the exhalation path member 120 or a position thereof is adjustable.

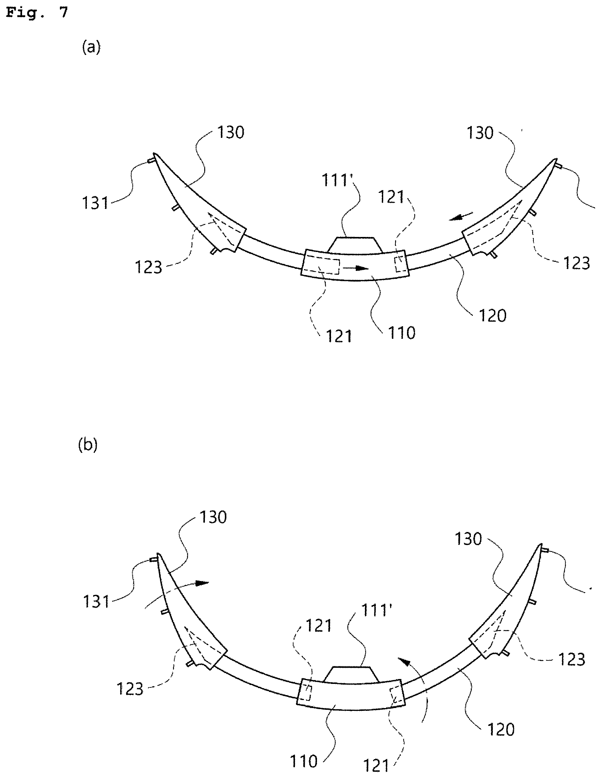

Therefore, the distance between the exhalation exhaust member 130 and the exhalation vent member 110 is adjustable, and thus, a position or angle of connection thereof may be adjusted to place the exhalation exhaust member 130 inside the regions A of FIG. 1 according to a shape or size of a user's face. This will be described with reference to FIG. 7 below.

FIG. 3C illustrates a breathing guide apparatus according to another embodiment of the present invention. As shown in FIG. 3C, both ends of the exhalation path member 120 may be provided with a ring-shaped bump 127 in a circumferential direction to prevent separation when the exhalation vent member 110 and the exhalation exhaust member 130 are fastened therewith or to seal the exhalation vent member 110 and the exhalation exhaust member 130.

The bump may be integrally formed with the exhalation path member 120 or may formed separately of a material such as rubber, resin or silicon.

The exhalation path member 120 may be formed of a polyvinyl chloride (PVC) material, polypropylene (PP), polyethylene (PE) or a low-density polyethylene (LDPE) material.

Similarly, the exhalation vent member 110 or the exhalation exhaust member 130 may be formed of the PVC material, PP, PE or the LDPE material.

Alternatively, the exhalation vent member 110, the exhalation path member 120, or the exhalation exhaust member 130 may be formed of silicon material for human body.

When PVC is applied, the use of plasticizers, known as including environmental hormones, for flexibility may be strictly limited.

In addition, an edge of the exhaust port 131 of the exhalation exhaust member 130 may be coated with an adhesive material to attach the inner circumferential surface of the filter unit 410 of the mask 400 thereto or seal the exhaust port 131.

The adhesive material is not intended to adhere the exhaust port 131 and the filter unit 410 not to be separated from each other, and thus, a degree of adhesion thereof is not limited, provided that a state of contact between the exhalation exhaust member 130 and the inner circumferential surface of the filter unit 410 of the mask 400 can be maintained even when shaken. The adhesive material may be a material such as a double-sided tape, a gel or sol type silicon, or the like.

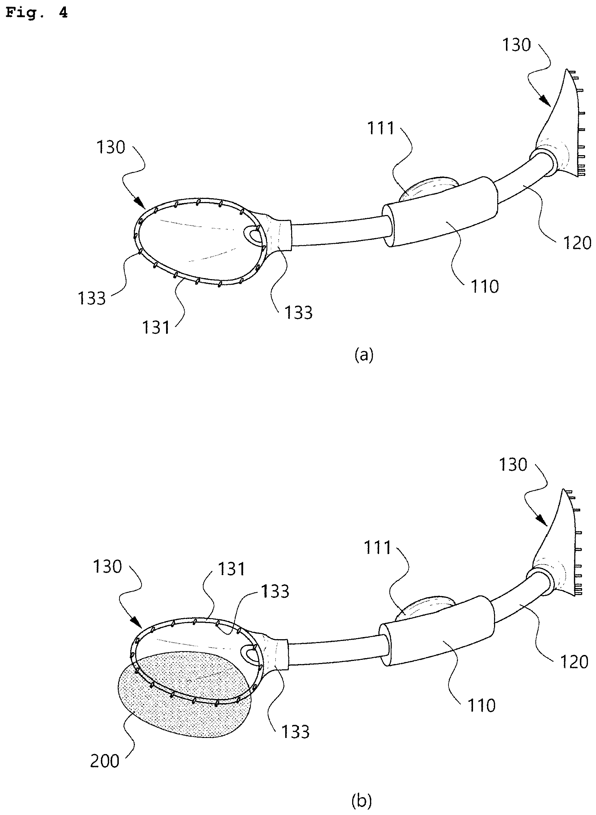

An embodiment illustrated in FIG. 4 shows an example in which the edge of the exhaust port 131 of the exhalation exhaust member 130 is provided with a plurality of fixing pins 133 as fixing means for fixing the inner circumferential surface of the filter unit 410 of the mask 400 thereon.

In the embodiment of FIG. 3, the edge of the exhaust port 131 of the exhalation exhaust member 130 is coated with the adhesive material, whereas in the embodiment of FIG. 4, the exhaust port 131 of the exhalation exhaust member 130 may be integrally formed with the plurality of fixing pins 133, as fixing means, which protrude toward the filter unit 410 of the mask 400.

Here, the fixing pins 133 may pass through the filter unit 410 to closely fix the filter unit 410 to the exhalation exhaust member 130. Here, the number, size, and shape of the fixing pins 133 may be freely determined, and, for example, the fixing pins 133 may be integrally formed with the exhaust port 131 and one end of each of them may be pointed to easily pass through the filter unit 410.

Although not shown in FIG. 4, an end of the fixing pin 133 may be configured in various shapes, such as a wedged or arrow shape, to easily pass through the filter unit 410 but be stopped in a direction in which the fixing pin 133 is inserted.

When the exhalation exhaust member 130 is formed of a PVC material, LDPE, or the like, the exhalation exhaust member 130 may have a certain level of flexibility and rigidity enough to pass through the filter unit 410 and not to be broken, when the fixing pin 133 integrally formed with the exhalation exhaust member 130 is sufficiently thin.

As shown in FIG. 4B, an exhaust port filter 200 may be further provided to filter exhalation exhausted through the exhaust port 131 of the exhalation exhaust member 130.

When the fixing pin 133 or the adhesive material is provided, the exhaust port filter 200 may be fixed to the exhaust port 131 in the same manner as the method of fixing the exhalation exhaust member 130 to the filter unit 410 of the mask 400.

Similar to the filter unit 410 of the mask 400, the exhaust port 200 may include an MB filter, a nano filter, an additional nonwoven filter, a functional filter, or a combination thereof, and may be formed in about one to five layers.

When the exhaust port filter 200 is added to the breathing guide apparatus 100 according to the present invention, filtering may be additionally performed, and the exhaust port filter 200 has a smaller area and a simpler structure than those of the mask 400. Therefore, when a package of a plurality of exhaust port filters 200, e.g., two to ten exhaust port filters 200, are provided together with the breathing guide apparatus 100 or the like, a user of the mask 400 may replace only an exhaust port filter 200, which is wet or contaminated, with another one and thus a period of use of the mask 400 having a relatively large area may be increased, thereby achieving an economic effect of minimizing costs associated with the mask 400.

The exhaust port filters 200 are provided to additionally filter exhalation without interfering with a path of inhalation and thus may have a shape and size corresponding to those of the exhalation exhaust member 130 of the breathing guide apparatus 100, and may have a size greater than that of the exhalation exhaust member 130 so that the fixing pin 133 may be easily mounted thereon.

When a user of the mask system has been infected with an infectious disease, the exhaust port filters 200 may help the user to breathe and alleviate the infection of the infectious disease to people nearby.

The exhaust port filters 200 may be put on the user's face to effectively filter fine dust, yellow dust, etc. introduced from the outside when the user breathes, and prevent pathogens contained in droplet particles from being exhausted to the outside of the filter unit 410 when the user coughs or sneezes, thereby promoting public hygiene.

The exhaust port filters 200 may be detachably attached to the breathing guide apparatus 100, and a mask replacement cycle may be increased by replacing only an exhaust port filter 200 in which moisture or pollutants contained in exhalation are intensively collected, thereby reducing costs.

An area of the exhaust port filter 200 may be larger than that of the exhalation exhaust member 130 of the breathing guide apparatus 100, and may be fixed in close contact with the inner circumferential surface of the filter unit 410 of the mask 400 through the fixing pins 133 to pass therethrough when mounted on the breathing guide apparatus 100.

The exhaust port filter 200 is applicable to almost all masks other than fine dust filtering masks and thus may be used by being attached to a general mask to prevent the spread of a disease, when there is a possibility of propagation of pathogens from exhalation of a person infected with or suspected to be infected with a virus disease.

To mount the exhaust port filter 200 on the exhalation exhaust member 130 or the exhaust port 131 of the breathing guide apparatus 100, not only a method of coating an edge of the exhaust port 131 of the exhalation exhaust member 130 with an adhesive material to attach or seal the inner circumferential surface of the filter unit 410 of the mask 400 but also a method of coating an edge of the exhaust port filter 200, i.e., a portion of the exhaust port filter 200 to be brought into contact with an edge of the exhaust port 131 of the exhalation exhaust member 130, with the adhesive material may be used. In this case, the exhaust port filter 200 may be provided in the form of a sticker to improve usability.

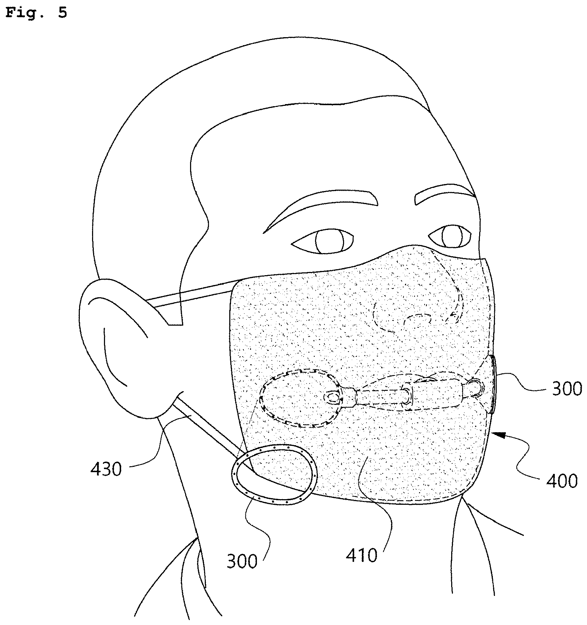

FIG. 5 illustrates a state in which a user is wearing a mask system equipped with a breathing guide apparatus according to another embodiment of the present invention.

The exhaust port 131 may be fixed to the filter unit 410 through a fixing member 300, as well as by allowing the fixing pin 133 to pass through the filter unit 410.

As shown in FIG. 5, the fixing member 300 with a fine fixing hole through which the fixing pin 133, when passing through the filter unit 410 and exposed via a front surface of the filter unit 410, passes through may be further provided. The fixing member 300 may be formed of an elastic material such as silicon or rubber and have a ring shape having an open central portion to correspond to the shape of the exhaust port 131 of the exhalation exhaust member 130. Accordingly, the filter unit 410 may be fixed in close contact between the exhalation exhaust member 130 and the fixing member 300, and minimize leakage of exhalation through a through-hole generated when the fixing pin 133 passes through the mask 400.

Further, the fixing member 300 may seal an exposed outer side of the fixing pin 133 passing through the filter unit 410 and thus prevent the skin or the like from being pricked by the fixing pin 133, thereby improving the convenience of use of the breathing guide apparatus 100.

In this case, the through-hole of the fixing member 300 may be configured in a through-groove to prevent the fixing pin 133 from protruding outside the fixing member 300.

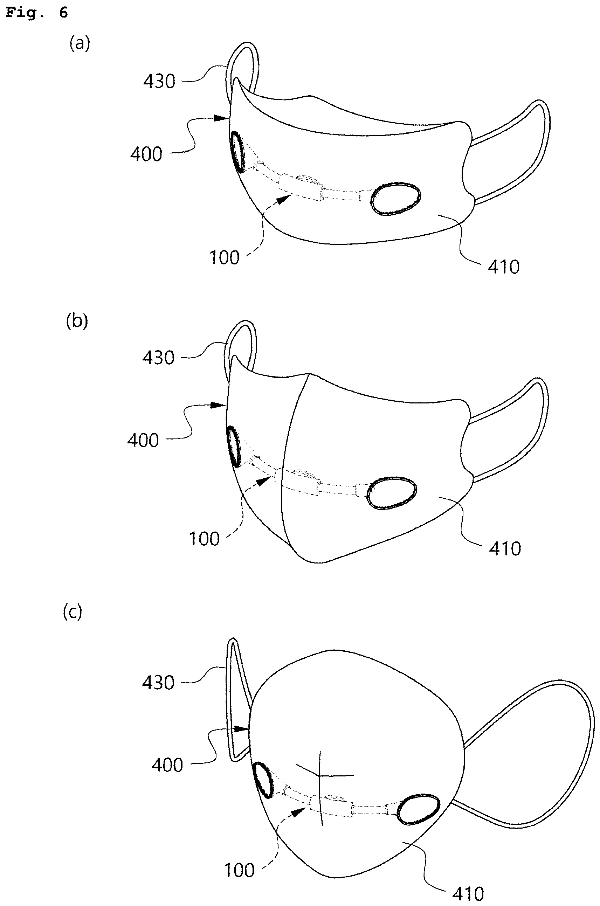

FIG. 6 illustrates various examples of a mask included in a mask system according to an embodiment of the present invention.

A breathing guide apparatus as described above with reference to FIGS. 1 to 5 is applicable to various types of masks. Generally, the breathing guide apparatus is applicable to masks capable of filtering fine dust and viruses, e.g., a mask 400 of FIG. 6A in which a filter unit 410 is folded in three or four folds in a vertical direction, a mask 400 of FIG. 6B in which the filter unit 410 is folded in two folds in a horizontal direction, and a mask 400 of FIG. 6C in which the filter unit 410 is not folded and is configured in a dome shape.

The masks 400 of FIGS. 6A and 6B may be manufactured by cutting a filter member into several pieces and bonding the pieces together and the mask 400 of FIG. 6C may be shaped in a mold but all of them are configured such that, when a user wears them, a region corresponding to the user's mouth protrudes upward but a region corresponding to the user's cheeks or ears comes in close contact with the skin.

Therefore, there is an advantage that an exhalation exhaust member 130 may exhaust exhalation while in close contact with the filter unit 410.

FIG. 7 illustrates a breathing guide apparatus according to another embodiment of the present invention.

Specifically, FIG. 7A illustrates a process of adjusting a whole width of a breathing guide apparatus by causing an outer end portion of an exhalation path member 120 to slide within an exhalation exhaust member 130. FIG. 7B illustrates a state in which an angle of connection of an inner end portion of the exhalation path member 120 in the exhalation vent member 110 changes.

In the embodiment of FIG. 7, an outer diameter of a vent port 111' of the exhalation vent member 110 may decrease toward ends thereof to minimize interference or contact with the lips of a user of a mask.

That is, edges of the vent port 111' are inclined inward in a plane view of FIG. 7, and the vent port 111' may have an oval, track, or circular shape, when observed in a direction toward the lips of a user of a mask.

When the outer diameter of the vent port 111' decreases toward the ends thereof, a user of a mask in which the breathing guide apparatus 100 is disposed is able to effectively exhale without making conscious effort to open his or her lips or bite the vent port 111' with his or her teeth or mouth, because the vent port 111' is naturally placed in front of the user's lips and interference or contact between the lips and the vent port 111' is minimized due to the decrease in the outer diameter toward the lips.

As shown in FIG. 7, the exhalation path member 120 may be in the form of a pipe of a resin material and be configured such that it slides within the exhalation vent member 110 and the exhalation exhaust member 130 and an angle of insertion thereof is adjustable to a certain extent.

Therefore, as shown in FIG. 7A, a whole width of the breathing guide apparatus may be adjusted by causing an inner end 121 and an outer end 123 of the exhalation path member 120 to slid within the exhalation vent member 110 and the exhalation exhaust member 110, and thus, a position of the exhalation exhaust member 130 may be adjusted according to the size or area of a user of a mask.

Referring to FIG. 7B, because an angle of connection of the inner end 121 or the outer end 123 of the exhalation path member 120 within the exhalation vent member 110 or the exhalation exhaust member 130 may be adjustable to a certain extent, the angle of connection may be adjusted according to various face shapes to prevent a filter unit of a mask from being lifted and to provide an optimal fit.

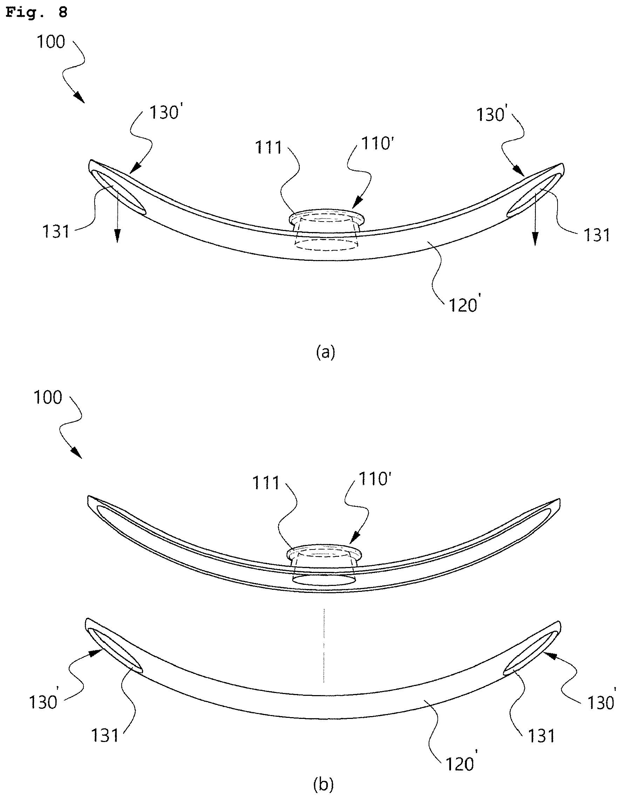

FIG. 8 illustrates a breathing guide apparatus according to another embodiment of the present invention.

In the case of the breathing guide apparatus described above, the exhalation vent member 110, the exhalation path member 120 and the exhalation exhaust member 130 may be configured independently and assembled together, whereas as illustrated in FIG. 8, an exhalation vent member, an exhalation path member, and an exhalation exhaust member are not separate components and a breathing guide apparatus may be partitioned into an exhalation vent member 110', an exhalation path member 120' and an exhalation exhaust member 130' and divided into two parts, i.e., a front housing and a rear housing, with respect to the exhalation path member 120'. When the front housing and the rear housing are assembled together, the breathing guide apparatus has a curved and slim pipe shape in which exhaust ports 131 are provided outward at both ends and a vent port 111 is provided inward at a center, as shown in FIG. 8A.

Similarly, the exhalation vent member 110' or the vent port 111 are illustrated as each having a cone shape, but embodiments of the present invention are not limited thereto and they may be configured in an inwardly recessed shape or a ridge-and-furrow form to minimize interference with a user's lips.

Alternatively, the exhalation vent member 110 'or the vent port 111 may be integrally formed with the rear housing or may be separately manufactured and mounted on the rear housing.

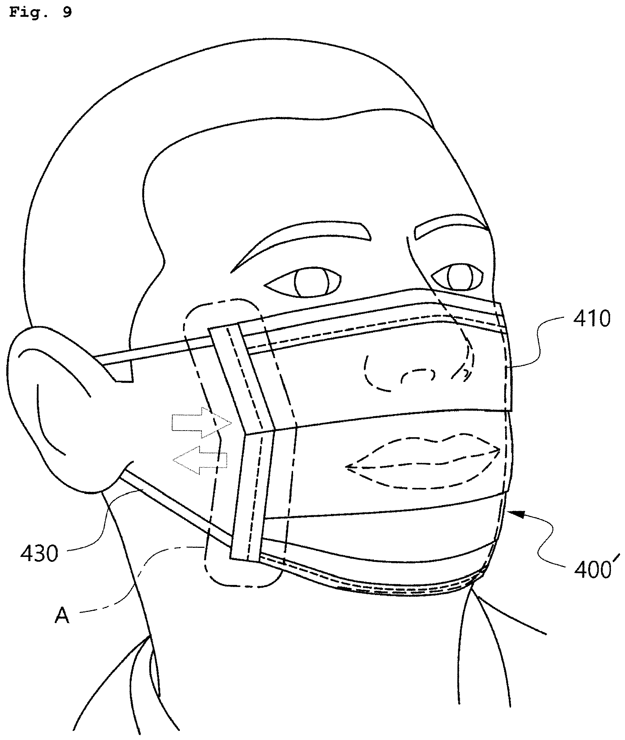

FIG. 9 illustrates a state in which a user is wearing a general disposable mask 400' mainly used for dental or medical purposes. The mask 400' of FIG. 9 may be manufactured by forming a filter unit 410 by corrugating one or more sheets of flat nonwoven fabric and connecting wearable members 430 to both ends of the filter unit 410, unlike the masks having three-dimensional (3D) shapes shown in FIG. 7.

Recently, masks have been used to mainly prevent infection of diseases due to pathogens or contagiousness viruses and thus it is recommended to frequently exchange a mask with new one to prevent infection of diseases.

Masks as shown in FIG. 9 are also appropriate to prevent infection due to droplets and are relatively cheap and thus are widely used.

However, unlike the masks having the 3D shapes of FIG. 7, the mask 400' of FIG. 9, which is formed of flat nonwoven fabric, is relatively thin and is not formed in a 3D shape and thus left and right end regions A thereof are more likely to be lifted when a user wears the mask 400'.

To prevent this problem, in the breathing guide apparatus 100 according to the present invention, the ring shape of the fixing member 300 of FIG. 5 may be changed to a cap or stopper shape so as to increase a degree of contact of the regions A with the skin.

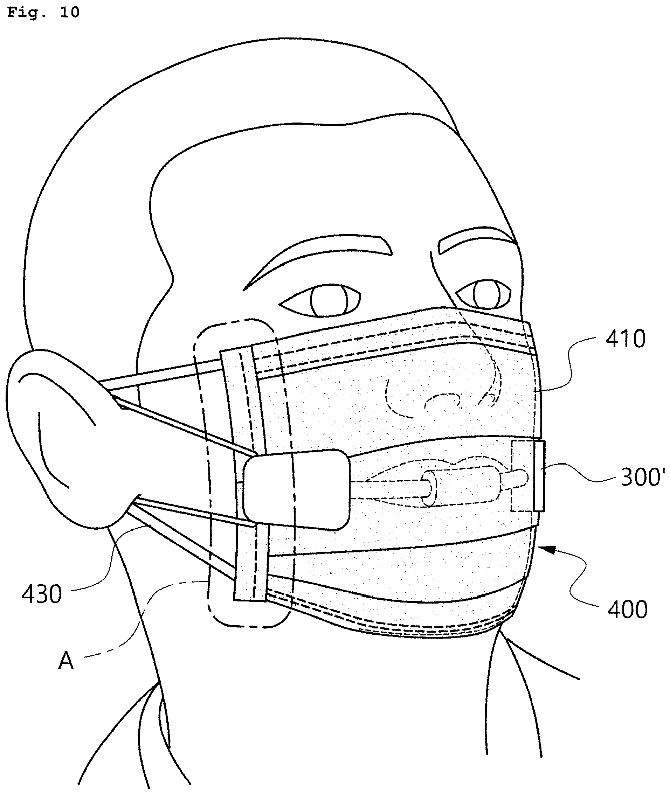

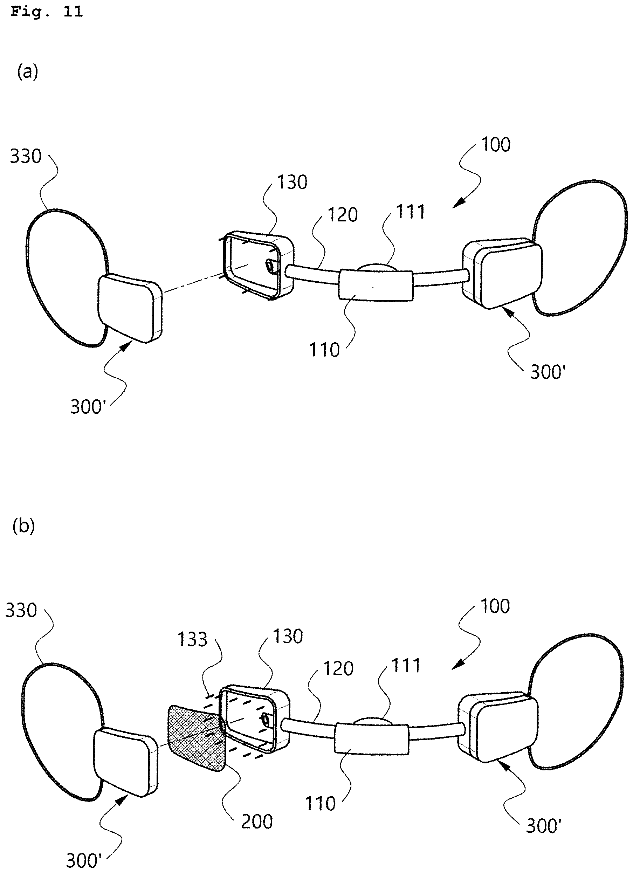

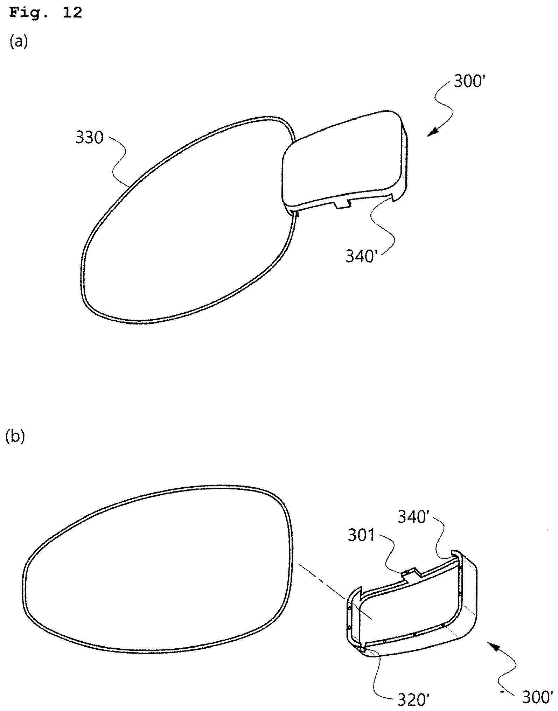

FIG. 10 is a perspective view of a state in which a breathing guide apparatus according to another embodiment of the present invention is mounted in the mask of FIG. 9. FIG. 11 illustrates a state in which a fixing member is separated from a breathing guide apparatus according to an embodiment of the present invention. FIG. 12 illustrates front and rear perspective views of a fixing member of a breathing guide apparatus according to an embodiment of the present invention.

A breathing guide apparatus 100 illustrated in FIG. 10 may further include a new type of a fixing member 300 to be mounted on an outer circumferential surface of a filter unit 410.

Referring to FIG. 5, the fixing member 300 is formed in a ring shape having an open central portion.

However, the fixing member 300' illustrated in FIGS. 10 to 12 is not limited to a ring shape, and may be embodied as a cap type member and may be provided with wearable bands 330.

That is, the fixing member 300' may be provided with the wearable bands 330 configured to be hung on a user's ears so as to prevent leakage of exhalation and inflow of non-filtered external air due to the lifting of the regions A.

As illustrated in FIGS. 11 and 12, the wearable bands 330 may be in the form of a flexible strip to be connected to the fixing member 300' and to be hung on the ears. The wearable bands 330 may be formed of the same material as the wearable member 430 of the mask 400'.

Therefore, as shown in FIG. 11, when a user of the mask 400' attaches the fixing member 330' to an outer side of the filter unit 410 and hangs, on his or her ears, the wearable bands 330 having an elastic and stretchable property and connected to the fixing member 330', both end regions A of the filter unit 410 are pressed to be less or not lifted.

The fixing member 300' may be provided with a wearable-band groove 30' to mount the wearable band 330 to the fixing member 300'.

Although a hole may be formed to mount the wearable bands 330 to the fixing members 300', the wearable-band groove 320' may be formed so that the wearable band 330 may be exchanged with new one when the wearable band 330 is contaminated. The wearable-band groove 320' may be inclined not to be easily separated even when tension is applied to the wearable band 330.

As shown in FIGS. 10 to 12, when the fixing member 300' is in the form of a cap, a plurality of exhaust ports 340' may be formed in an edge region of the fixing member 300'.

That is, when exhalation passing through the exhalation exhaust member 130 and the filter unit 410 of the mask 400' is exhausted in a direction perpendicular to the filter unit 410, condensation may be generated on glasses or the like due to moisture contained in the exhalation according to weather. To solve this problem, the fixing member 300' may be formed in the form of a cap such that exhalation may be exhausted to the exhaust ports 340' on lateral sides of the edge region other than upper and lower sides of the edge region.

Similar to the embodiments of FIGS. 4 and 5, a fixing hole into which a fixing pin 133 is insertable may be provided on an inner circumferential surface of an edge of the fixing member 300' which is in the form of the cap.

The fixing pin 133 may be integrally formed with the exhalation exhaust member 130 by injection molding or the like but may be formed of a metal or resin material, manufactured separately from the exhalation exhaust member 130, and mounted on the exhalation exhaust member 130 by pressing-in or insert-injection molding to stably support the fixing member 300' which is in the form of the cap.

As illustrated in FIG. 11, the fixing member 300', which is in the form of the cap, may further be provided with an exhaust port filter 200, thereby reducing a risk of the spread of a disease even when a user of a mask is a patient or an infected person.

Therefore, when the fixing member 300' equipped with the wearable band 330 is applied, a filter unit of even a corrugated nonwoven fabric mask may be prevented from being lifted, thereby minimizing a risk of inflow of external air that does not pass through the filter unit or leakage of exhalation which does not pass through the fixing part.

The fixing members 300' provided with the wearable bands 330 described above with reference to FIGS. 10 to 12 are applicable to various types of masks as illustrated in FIG. 7, as well as a corrugated nonwoven fabric mask.

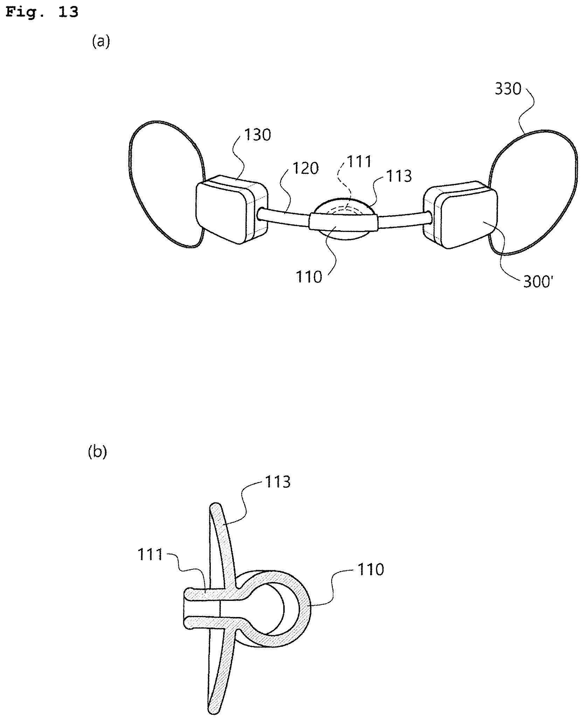

FIG. 13 illustrates a breathing guide apparatus according to another embodiment of the present invention. Specifically, FIG. 13A is a top view of the breathing guide apparatus, and FIG. 13B is a cross-sectional view of an exhalation vent member 110 illustrated in FIG. 13A, according to an embodiment of the present invention. A description of parts of FIG. 13 that are the same as those of the above description is omitted here.

The embodiment of FIG. 13 is an example in which the exhalation vent member 110 further includes a blocking plate 113, unlike in the above-described embodiments.

The exhalation vent member 110 may include a chamber type body. The exhalation vent member 110 may include: a vent port 111 configured to be disposed in front of the mouth of a user of a mask, at least one exhaust port for delivering exhalation exhausted via the vent port 111 to an exhalation path member 120, and a blocking plate 113 for preventing contact between a filter unit of the mask and the user's mouth.

The blocking plate 113 is a blocking means for preventing contact between the user's lips, which are likely to be stained with saliva, and the filter unit of the mask, which is likely to be contaminated with foreign substances, bacteria or viruses.

The filter unit of the mask is configured to filter exhalation and thus is likely to be always exposed to moisture, and is continuously exposed to saliva, when in contact with the lips. Thus, the filter unit 410 is a region in which bacteria is likely to breed or which is likely to be contaminated with foreign substances.

Accordingly, the exhalation vent member 110 may be provided with the blocking plate 113 to prevent the lips from being in contact with a filter unit of a mask, so that bacteria and the like penetrating the filter unit may be prevented from flowing to the inside of the mouth through the lips and the filter unit may be prevented from being contaminated due to frequent contact between the lips and the filter unit of the mask.

As illustrated in FIG. 13B, the blocking plate 113 may be provided between the body of the exhalation vent member 110 and the vent port 111 or provided in front of the body of the exhalation vent member 110 to be in contact with an inner circumferential surface of a filter unit of a mask.

While the present invention has been described above with respect to exemplary embodiments thereof, it would be understood by those of ordinary skilled in the art that various changes and modifications may be made without departing from the technical conception and scope of the present invention defined in the following claims. Thus, it is clear that all modifications are included in the technical scope of the present invention as long as they include all the components as claimed in the claims of the present invention.

## Claims

1. A breathing guide apparatus mounted inside a filter unit of a mask for air filtration, the breathing guide apparatus comprising:
an exhalation vent member configured to be disposed in front of the mouth of a user of the mask to exhaust an exhalation from the user's mouth;
at least one exhalation path member connected to the exhalation vent member and extending to a left or right side of the exhalation vent member; and
at least one exhalation exhaust member configured to cause the exhalation to pass through the inner circumferential surface of the filter unit, filter foreign substances contained in the exhalation, and exhaust the resultant exhalation, the at least one exhalation exhaust member being provided on an end of the exhalation path member to be in close contact with an inner circumferential surface of the filter unit,
wherein the exhalation exhaust member is brought in close contact with a left or right inner circumferential surface of the filter unit of the mask to cause the user's inhalation to pass through the filter unit of the mask and be inhaled into the user's nose and to cause the user's exhalation to pass through the filter unit of the mask and be exhausted without being mixed with the inhalation.

2. The breathing guide apparatus of claim 1, wherein a pair of exhalation path members and a pair of exhalation exhaust members are provided to be connected to the left side and a right side of the exhalation vent member.

3. The breathing guide apparatus of claim 1 or 2, wherein the exhalation exhaust member is provided in the form of a concave bowl, and the exhalation path member is connected to a lateral side of the exhalation exhaust member and wherein the exhalation path member is mounted to pass through the exhalation exhaust member.

4. The breathing guide apparatus of claim 3, wherein an end of the exhalation path member is inclined to prevent interference with the inner circumferential surface of the filter unit of the mask, while being mounted to pass through the exhalation exhaust member.

5. The breathing guide apparatus of claim 3 or 4, wherein the exhalation exhaust member tapers in a direction opposite to a connection portion thereof to which the exhalation path member is connected.

6. The breathing guide apparatus of claim 5, wherein an exhaust port of the exhalation exhaust member is curved backward to be in close contact with the filter unit of the mask.

7. The breathing guide apparatus of one of claims 3 to 6, wherein both ends of the exhalation path member are provided with ring-shaped bumps in a circumferential direction to prevent separation of the exhalation path member or seal the exhalation path member, when fastened with the exhalation vent member and the exhalation exhaust member.

8. The breathing guide apparatus of one of claims 1 to 7, wherein an edge of an exhaust port of the exhalation exhaust member is provided with a plurality of fixing pins to fix the inner circumferential surface of the filter unit of the mask.

9. The breathing guide apparatus of one of claims 1 to 8, wherein the exhalation vent member comprises:
a vent port configured to be placed in front of the user's mouth; and
at least vent port configured to deliver an exhalation vent through the vent port to the exhalation path member.

10. The breathing guide apparatus of one of claims 1 to 9, wherein at least a region of the exhalation path member has a height greater than a thickness of a cross section thereof.

11. The breathing guide apparatus of claim 3 or one of claims 4 to 10 in combination with claim 3, wherein the exhalation path member is mounted in the exhalation vent member or the exhalation exhaust member to be insertable into or withdrawable from the exhalation vent member or the exhalation exhaust member, and thus, a distance between the exhalation exhaust member and the exhalation vent member and an angle of connection to the exhalation path member are adjustable.

12. The breathing guide apparatus of one of claims 1 to 11, wherein the exhaust port of the exhalation exhaust member is further provided with a plurality of fixing pins to pass through the filter unit of the mask, and
the breathing guide apparatus further comprises a fixing member formed in the form of a ring or a cap provided with a fixing hole or a fixing groove into which the fixing pins, which pass through the filter unit of the mask and protrude from a front surface of the filter unit, are inserted and fixed.

13. The breathing guide apparatus of claim 12, further comprising wearable bands configured to be mounted on the fixing member to be hung on the user's ears, when the fixing member is formed in the form of the cap.

14. The breathing guide apparatus of claim 12 or 13, wherein, when the fixing member is formed in the form of the cap, an exhaust port is formed on a lateral side or a lower side of an edge of the fixing member.

15. The breathing guide apparatus of one of claims 1 to 14, wherein the exhalation vent member has a chamber type body, wherein the exhalation vent member comprises:
a vent port configured to be disposed in front of a mouth of a user of the mask;
at least one exhaust port configured to deliver exhalation exhausted via the vent port to the exhalation path member; and
a blocking plate configured to prevent contact between the filter unit of the mask and the user's lips.
